Europäisches Patentamt

European Patent Office

Office européen des brevets

(11) Publication number: **0 103 995**
**B1**

(12) **EUROPEAN PATENT SPECIFICATION**

(45) Date of publication of patent specification: **11.04.90**

(51) Int. Cl.⁵: **A 61 K 9/02**

(21) Application number: **83304847.3**

(22) Date of filing: **23.08.83**

(54) Medicated suppository.

(30) Priority: **24.08.82 US 411123**

(43) Date of publication of application:
**28.03.84 Bulletin 84/13**

(45) Publication of the grant of the patent:
**11.04.90 Bulletin 90/15**

(84) Designated Contracting States:
**AT BE CH DE FR IT LI LU NL SE**

(56) References cited:
**DE-A-2 530 563**
**FR-A-2 443 244**
**GB-A- 843 886**

**CHEMICAL ABSTRACTS, vol. 94, no. 12, June 1981, page 357, no. 197573j, Columbus, Ohio, US; & JP - A - 81 25 109 (DIA SEIYAKU Y.K.) 10-03-1981**

**CHEMICAL & PHARMACEUTICAL BULLETIN, vol. 30, no. 8, August 1982, pages 2900-2905; SHUN'ICHI NORO et al.: "Studies on pharmaceutical drug design for suppositories. I. Effect of physico-chemical properties of surfactants and polymers on emulsion-type bases"**

(73) Proprietor: **CILAG AG**
**Hochstrasse 201/9**
**CH-8201 Schaffhausen (CH)**

(72) Inventor: **Niederer, Roland Rudolf**
**IM Landgut**
**CH-8211 Stetten (CH)**
Inventor: **Zulliger, Hans Walter**
**IM Obstgarten**
**CH-8450 Andelfingen (CH)**

(74) Representative: **Jones, Alan John et al**
**CARPMAELS & RANSFORD**
**43 Bloomsbury Square**
**London, WC1A 2RA (GB)**

(56) References cited:

**CHEMICAL ABSTRACTS, vol. 85, no. 22, 29th November 1976, page 443, no. 166595c, Columbus, Ohio, US; A.MOES: "Effects of the addition of aluminum stearate and colloidal silica on the properties of suppository formulations" & J. PHARM. BELG. 1976, 31(4), 355-66**

Courier Press, Leamington Spa, England.

## Description

This invention relates to a medicated suppository for vaginal or rectal application. In particular, the invention relates to a medicated suppository capable of releasing the contained medicament evenly over the walls of the vaginal or rectal cavity. The invention is a triglyceride based suppository and is additionally comprised of a medicament, a gel forming agent and a gel dispersing agent.

The administration of medicaments in the form of suppositories is known in the art. In general usage, however, it has been found that upon liquefaction of the suppository some of the fluid containing the medicament flows out of the vaginal or rectal cavity leaving less of the medicament available for the intended therapeutic use. To counteract this loss of medicament, higher concentrations of the medicament are sometimes employed. As a result, the patient is exposed to higher doses of the medicament than are actually required for successful treatment. In addition, the cost of the therapy is of necessity higher due to the use of excess medicament.

DE—A—2 530 563 relates to the preparation of enteral compositions containing addictive drugs which are less open to misuse. Among the enteral preparations described are suppositories containing propiram fumarate, methyl cellulose or traganth powder, and hard fat.

FR—A—2 443 244 relates to the preparation of suppositories for enabling the sustained release of medicaments. The suppositories comprise a fatty acid glyceride having a fusion point of at least 37°C, a low viscosity, non-irritant organic substance, a water swellable organic polymer and a surfactant.

According to a first aspect of the present invention there is provided a medicated suppository comprising:

at least 60% by weight of a mixture of triglycerides of the fatty acids $C_{10}H_{20}O_2$ to $C_{18}H_{30}O_2$;

5 to 25% by weight of polygum, guar gum, an alkali metal or alkaline metal salt of alginic acid, polygel or xanthan gum as a gel forming agent;

4 to 8% by weight of stearyl heptanoate, purcelline oil, a partial fatty acid glycerol ester or a polyoxyethylene sorbitan fatty acid ester as a gel dispersing agent; and

4 to 15% by weight of a medicament, in which the amounts of the ingredients of the suppository must always add up to 100% by weight.

According to a second aspect of the present invention there is provided a suppository base comprising:

at least 60% by weight of a mixture of triglycerides of the fatty acids $C_{10}H_{20}O_2$ to $C_{18}H_{30}O_2$;

5 to 25% by weight of polygum, guar gum, an alkali metal or alkaline metal salt of alginic acid, polygel or xanthan gum as a gel forming agent; and

4 to 8% by weight of stearyl heptanoate, purcelline oil, a partial fatty acid glycerol ester or a polyoxyethylene sorbitan fatty acid ester as a gel dispersing agent,

in which the amounts of the ingredients of the suppository base must always add up to 100% by weight.

The medicated suppository of the present invention is comprised of a triglyceride as the carrier, a gel forming agent and a gel dispersing agent in combination with a medicament. The desired therapeutic effect is achieved by a diffusion of the medicament throughout the vaginal or rectal cavity by means of the dispersing agent and the adhesive properties of the gel forming agent which combine to prevent the liquified suppository from flowing out of the vaginal or rectal cavity. The availability of the medicament is thus increased by the resultant increase of the surface of absorption and the extension of the time during which the medicament resides in the vaginal or rectal cavity The unique combination of ingredients results in a homogeneous composition which is capable of distributing the medicament throughout the vaginal or rectal cavity. This results in the administration of smaller doses of the medicament than those required in conventional suppositories.

Mixtures of mono-, di- and triglycerides of the fatty acids $C_{10}H_{20}O_2$ to $C_{18}H_{30}O_2$ (hard fat, commonly known in the art as Adeps solidus) may be employed as the carrier.

The medicated suppository may contain one or more of a number of known medicaments including: antibiotics such as tetracycline hydrochloride, erythromycin, neosporin, achromycin and chloromycetin; antimycotics such as econazole nitrate, miconazole nitrate and clotrimazole; anti-inflammatory agents such as aspirin, clocortolone pivalate, hydrocortisone, tolmetin sodium and indomethacin; estrogens; trichomonacids; and disinfectants. The amount of medicament present in the suppository will depend upon the particular medicament employed.

In certain formulations better homogenicity may be obtained by the use of a stabilizer for the colloidal suspension. Colloidal silicon dioxide and urea can be used as the stabilizing agent. In those cases where the medicament is only moderately soluble in the suppository formulation, a solubilizing agent such as sorbitan monostearate or polyethylene glycol may be employed. With certain medicaments, it may be necessary to employ a preservative such as benzoic acid or an antioxidant such as butylated hydroxytoluene in the formulation.

The suppository of the present invention is generally prepared by first mixing the gel forming agent and the medicament in a suitable vessel and micronizing the mixture. The medicament is then added in portions; after each addition the granules are pressed through a sieve and the mixing is continued for several minutes. In a separate vessel the fatty acid triglyceride and the gel dispersing agent are melted together at a temperature of about 50—60°C. The melt is cooled to about 40°C. The two mixtures are combined with stirring and the combined mixture is then homogenized for about 10 minutes at a temperature between 38—40°C. The mixture is then cooled to about 36—39°C and placed in a suppository shell. If a stabilizing agent is to be employed in the suppository, it is generally added to the mixture of the fatty acid triglyceride and the gel dispersing agent after the melt has been cooled. The solubilizing agents and preservatives, when employed, can be added at various stages during the procedure.

Although the suppository of the present invention may be prepared in a variety of shapes, the preferred shape is an egg-shaped ovule. The suppository preferably has a weight between 1 and 3 g. The preferred weight is about 2.2—2.7 g.

A more detailed description of the process according to the invention is described in Procedure 1.

Procedure 1

A. Polygel (24 Kg) is placed in a mixing vessel. Econazole nitrate (8 Kg) is added in portions and the mixture is micronized and mixed for 5 minutes. After each addition of econazole nitrate, the granules are pressed through an 0.4 mm sieve and then mixed for 10 minutes.

B. In a separate vessel adeps solidus (Witepsol® H19; 64.72 Kg), adeps solidus (Wecobee® FS; 269.18 Kg) and stearyl heptanoate (21.82 Kg) are added together and the mixture is melted at 50—60°C. The melt is cooled to 40°C, colloidal silicon dioxide (4.32 Kg) is added and the mixture is homogenized for 5 minutes. This mixture is then combined with the mixture prepared in A above with stirring and the resulting mixture is homogenized for 10 minutes at 38—40°C under vacuum. The homogenate is then cooled to 36—39°C *in vacuo* and placed in a multiplast container.

The following are examples of suppositories falling within the present invention.

### Example 1

| | mg/suppository |
|---|---|
| Econazole nitrate | 150.0 |
| Polygel | 300.0 |
| Colloidal silicon dioxide | 27.0 |
| Adeps solidus (Witepsol® H19 | 404.2 |
| Adeps solidus (Wecobee® FS) FS) | 1682.4 |
| Stearyl heptanoate | 136.4 |

### Example 2

| | mg/suppository |
|---|---|
| Metronidazole | 350.0 |
| Alginate | 350.0 |
| Colloidal silicon dioxide | 27.0 |
| Adeps solidus (Novata® 299) | 413.0 |
| Adeps solidus (Wecobee® FS) | 1410.0 |
| Softigen 701 | 150.0 |

### Example 3

| | mg/suppository |
|---|---|
| Estriol | 0.5 |
| Benzoic acid | 0.8 |
| Butylated hydroxytoluene | 0.5 |
| Polygel | 300.0 |
| Polyethylene glycol 400 | 60.0 |
| Polyethylene glycol 1000 | 300.0 |
| Sorbitan monostearate (Span® 60) | 215.0 |
| Adeps solidus (Witepsol® S55) | 1823.2 |

### Example 4

| | mg/suppository |
|---|---|
| Sulfathiazole | 170.0 |
| Sulfacetamide | 145.0 |
| Sulfabenzamide | 180.0 |
| Urea | 32.0 |
| Polygel | 300.0 |
| Adeps solidus (Witepsol® H12) | 1873.0 |

### Example 5

| | mg/suppository |
|---|---|
| Chlorquinaldol | 200.0 |
| Oxychinolin sulfate | 10.0 |
| Polygum (Guar) | 350.0 |
| Colloidal silicon dioxide | 25.8 |
| Adeps solidus (Witepsol® H19) | 429.7 |
| Adeps solidus (Wecobee® FS) | 1547.0 |
| Stearyl heptanoate | 137.5 |

The suppositories prepared according to the above examples liquefy in body fluids in less than thirty minutes at about 37°C.

The above examples are provided by way of illustration and are not meant to limit the scope of the present invention, which is defined by the appended Claims.

**Claims**

1. A medicated suppository comprising:

at least 60% by weight of a mixture of triglycerides of the fatty acids $C_{10}H_{20}O_2$ to $C_{18}H_{30}O_2$;

5 to 25% by weight of polygum, guar gum, an alkali metal or alkaline metal salt of alginic acid, polygel or xanthan gum as a gel forming agent;

4 to 8% by weight of stearyl heptanoate, percelline oil, a partial fatty acid glycerol ester or a polyoxyethylene sorbitan fatty acid ester as a gel dispersing agent; and

4 to 15% by weight of a medicament

in which the amounts of the ingredients of the suppository must always add up to 100% by weight.

2. The suppository of Claim 1, wherein the medicament is econazole nitrate, miconazole nitrate or clotrimazole.

3. The suppository of Claim 1 or Claim 2, further including a stabilizer.

4. The suppository of Claim 3, wherein the stabilizer is colloidal silicon dioxide.

5. The suppository of any one of Claims 1 to 4, which comprises:

14.97% by weight of adeps solidus (Witepsol® H19);

62.31% by weight of adeps solidus (Wecobee® F5);

11.11% by weight of polygel;

5.05% by weight of stearyl heptanoate;

5.56% by weight of econazole nitrate; and 1.0% by weight of colloidal silicon dioxide.

6. The suppository of any one of Claims 1 to 5, which has a weight of from 1 to 3 g.

7. A suppository base comprising:

at least 60% by weight of a mixture of triglycerides of the fatty acids $C_{10}H_{20}O_2$ to $C_{18}H_{30}O_2$;

5 to 25% by weight of polygum, guar gum, an alkali metal or alkaline metal salt of alginic acid, polygel or xanthan gum as a gel forming agent; and

4 to 8% by weight of stearyl heptanoate, purcelline oil, a partial fatty acid glycerol ester or a polyoxyethylene sorbitan fatty acid ester as a gel dispersing agent,

in which the amounts of the ingredients of the suppository base must always add up to 100% by weight.

8. The suppository of Claim 7, further including a stabiliser.

9. The suppository of Claim 8, wherein the stabilizer is colloidal silicon dioxide.

10. A process of preparing the medicated suppository any one of Claims 1 to 6, comprising mixing said ingredients in said proportions, and shaping the composition thus formed.

11. A process of preparing the suppository base of any one of claims 7 to 9, comprising mixing said ingredients in said proportions.

**Patentansprüche**

1. Ein medizinisches Zäpfchen umfassend:

wenigstens 60 Gew.-% eines Gemisches von Triglyzeriden der Fettsäuren $C_{10}H_{20}O_2$ bis $C_{18}H_{30}O_2$;

5 bis 25 Gew.-% Polygummi, Guargummi, eines Alkalimetallsalzes oder alkalischen Metallsalzes der Alginsäure, Polygel oder Xanthangummi als gelbindendes Mittel;

4 bis 8 Gew.-% Stearylheptanoat, Purcellinöl, partiellen Fettsäureglyzerinester oder Polyoxyethylensorbitanfettsäureester als geldispergierendes Mittel; und

4 bis 15 Gew.-% eines Medikamentes,

worin die Mengen der Bestandteile des Zäpfchens sich jeweils auf 100 Gew.-% ergänzen müssen.

2. Das Zäpfchen nach Anspruch 1, worin das Medikament Econazolnitrat, Micronazolnitrat oder Clotrimazol ist.

3. Das Zäpfchen nach Anspruch 1 oder 2, das weiterhin einen Stabilisator einschließt.

4. Das Zäpfchen nach Anspruch 3, worin der Stabilisator kolloidales Siliziumdioxid ist.

5. Das Zäpfchen nach einem der Ansprüche 1 bis 4, welches umfaßt:

14,97 Gew.-% Adeps Solidus (Witepsol® H19);

62,31 Gew.-% Adeps Solidus (Wecobee® F5);

11,11 Gew.-% Polygel;

5,05 Gew.-% Stearylheptanoat;

5,56 Gew.-% Econazolnitrat; und

1,0 Gew.-% kolloidales Siliziumdioxid.

6. Das Zäpfchen nach einem der Ansprüche 1 bis 5, welches ein Gewicht von 1 bis 3 g aufweist.

7. Eine Zäpfchenbasis, umfassend:

wenigstens 60 Gew.-% eines Gemisches aus Triglyzeriden der Fettsäuren $C_{10}H_{20}O_2$ bis $C_{18}H_{30}O_2$;

5 bis 25 Gew.-% Polygummi, Guargummi, eines Alkalimetallsalzes oder alkalischen Metallsalzes der Alginsäure, Polygel oder Xanthangummi als ein gelbildendes Mittel; und

4 bis 8 Gew.-% Stearylheptanoat, Purcellinöl, partiellen Fettsäureglyzerinester oder Polyoxyethylensorbitanfettsäureester als geldispergierendes Mittel,

worin die Mengen der Bestandteile der Zäpfchenbasis sich jeweils auf 100 Gew.-% ergänzen müssen.

8. Die Zäpfchenbasis nach Anspruch 7, welche weiterhin einen Stabilisator enthält.

9. Die Zäpfchenbasis nach Anspruch 8, worin der Stabilisator kolloidales Siliziumdioxid ist.

10. Ein Verfahren zur Herstellung des medizinischen Zäpfchens nach einem der Ansprüche 1 bis 6, umfassend das Vermischen der angegebenen Bestandteile in den angegebenen Verhältnissen und das Formgeben der solcherart gebildeten Zusammensetzung.

11. Ein Verfahren zur Herstellung der Zäpfchenbasis nach einem der Ansprüche 7 bis 9, umfassend das Vermischen der angegebenen Bestandteile in den angeführten Verhältnissen.

**Revendications**

1. Suppositoire médicinal comprenant:

au moins 60% en poids d'un mélange de triglycérides des acides gras $C_{10}H_{20}O_2$ à $C_{18}H_{30}O_2$;

5 à 25% en poids de polygomme, de gomme guar, d'un sel de métal alcalin ou de métal alcalino-terreux d'acide alginique, de polygel ou de gomme xanthanne, comme agent formant un gel;

4 à 8% en poids d'heptanoate de stéaryle, d'huile purcelline, d'un ester partiel de glycérol et d'acide gras ou d'un ester d'acide gras et de sorbitanne polyéthoxylé, comme agent dispersant les gels; et

4 à 15% en poids d'un médicament,

dans lequel les quantités des ingrédients du suppositoire doivent toujours faire au total 100% en poids.

2. Suppositoire selon la revendication 1, dans lequel le médicament est le nitrate d'éconazole, le nitrate de miconazole ou le clotrimazole.

3. Suppositoire selon la revendication 1 ou la revendication 2, contenant en outre stabilisant.

4. Suppositoire selon la revendication 3, dans lequel le stabilisant est de dioxyde de silicium colloïdal.

5. Suppositoire selon l'une quelconque des revendications 1 à 4, qui comprend:

14,97% en poids de lard solide (Witepsol® H19);
62,31% en poids de lard solide (Wecobee® F5);
11,11% en poids de polygel;
5,05% en poids d'heptanoate de stéaryle;
5,56% en poids de nitrate d'éconazole; et
1,0% en poids de dioxyde de silicium colloïdal.

6. Suppositoire selon l'une quelconque des revendications 1 à 5, qui a un poids de 1 à 3 g.

7. Base de suppositoire comprenant:

au moins 60% en poids d'un mélange de triglycérides des acides gras $C_{10}H_{20}O_2$ à $C_{18}H_{30}O_2$;

5 à 25% en poids de polygomme, de gomme guar, d'un sel de métal alcalin ou de métal alcalino-terreux d'acide alginique, de polygel ou de gomme xanthanne, comme agent formant un gel; et

4 à 8% en poids d'heptanoate de stéaryl, d'huile purcelline, d'un ester partiel de glycérol et d'acide gras ou d'un ester d'acide gras et de sorbitanne polyéthoxylé, comme agent dispersant les gels,

dans laquelle les quantités des ingrédients de la base de suppositoire doivent toujours faire au total 100% en poids.

8. Suppositoire selon la revendication 7 contenant en outre un stabilisant.

9. Suppositoire selon la revendication 8, dans lequel le stabilisant est le dioxyde de silicium colloïdal.

10. Procédé de préparation d'un suppositoire médicinal selon l'une quelconque des revendications 1 à 6, comprenant le mélange desdits ingrédients en lesdites proportions, et la mise en forme de la composition ainsi formée.

11. Procédé de préparation d'une base de suppositoire selon l'une quelconque des revendications 7 à 9, comprenant le mélange desdits ingrédients en lesdites proportions.